Europäisches Patentamt
European Patent Office
Office européen des brevets

(19)

(11) Publication number: **0 309 120 A2**

(12) # EUROPEAN PATENT APPLICATION


(21) Application number: **88308280.2**

(22) Date of filing: **07.09.88**

(51) Int. Cl.4: **C07K 7/10 , A61K 37/24 , G01N 33/74**

(30) Priority: **23.09.87 US 100047**
**24.06.88 CA 570432**

(43) Date of publication of application: **29.03.89 Bulletin 89/13**

(84) Designated Contracting States: **CH DE FR GB IT LI SE**



(71) Applicant: **QUEEN'S UNIVERSITY AT KINGSTON**

**Kingston Ontario K7L 3N6(CA)**

(72) Inventor: **Flynn, Thomas G.**
**5 Lakeland Point Drive**
**Kingston Ontario(CA)**
Inventor: **Jennings, Donald B.**
**153 Falkirk Terrace**
**Kingston Ontario(CA)**

(74) Representative: **Wood, Anthony Charles et al**
**Urquhart-Dykes & Lord 91 Wimpole Street**
**London W1M 8AH(GB)**


(54) **Atrial hypotensive peptide.**


(57) An atrial natriuretic peptide having profound natriuretic, diuretic and hypotensive effects, characterized by a 46 amino acid residue sequence:
Ser-Gln-Asp-Ser-Ala-Phe-Arg-Ile-Gln-Glu-Arg-Leu-Arg-Asn-Ser-Lys-Met-Ala-His-Ser-Ser-Ser-Cys-Phe-Gly-Gln-Lys-Ile-Asp-Arg-Ile-Gly-Ala-Val-Ser-Arg-Leu-Gly-Cys-Asp-Ile-Leu-Leu-Ile-Ala-Gln,
having a disulphide bond between the cysteine residues which may be extracted from mammalian heart atria is described. A synthetic 30 amino acid residue peptide corresponding to residues 17-46 of the above sequence has also been prepared and shown to have similar biological activity.


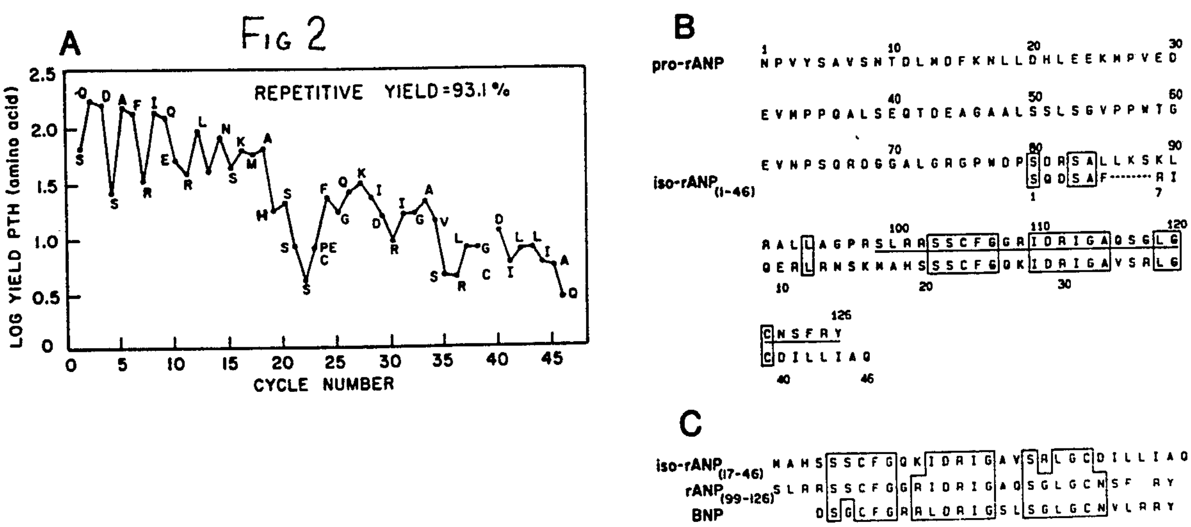


EP 0 309 120 A2

## Field of Invention

This invention relates to novel atrial hypotensive peptides and diagnostic kits therefor.

## Background of Invention and Description of Prior Art

Since the description by deBold et al, Life Sci 28 89-94 (1981) of a potent atrial natriuretic factor extracted from rat atria, considerable attention has been directed to a family of diuretic and natriuretic peptides which have been shown to be secreted in mammalian atria. Attention is directed to deBold U.S. Patent 4,663,437 issued 5 May 1987, Thibault et al U.S. Patent 4,607,023 issued 19 August 1986 and 4,455,864 issued 29 January 1985 and 10 December 1985 respectively. These references all teach atrial peptides having up to 71 amino acid residues in a sequence which always includes: Ser-Leu-Arg-Arg-Ser-Ser-Cys-Phe-Gly-Gly-Arg-Ile-Asp-Arg-Ile-Gly-Ala-Gln-Ser-Gly-Leu-Gly-Cys-Asn-Ser-Phe-Arg-Tyr or minor variations thereof depending on the atrial source (rat, human, etc.) and the precise method of isolation thereof. DeBold also established the existence of a disulphide bond between the cysteine residues and consequently the looped structure of the peptide. Recent sequence determinations by Needleman et al have shown (Science 1985; 229:397-400) the 28 amino acid peptide to be the circulating form of the peptide in humans. The human 28 amino acid peptide differs from the rat sequence only at position 110 at which position methionine occurs in the human peptide and isoleucine occurs in the rat peptide. These peptides, variously named ANF, cardionatrin, auriculin or atriopeptin, depending upon the authority, have been shown to exhibit profound diuretic, natriuretic and hypotensive effects of relatively short duration when injected into non-diuretic mammalian subjects, including man. These peptides are, therefore, of considerable interest and utility for therapeutic applications despite the lack of oral bioavailability and short half life of currently known analogs.

Initially ANF was produced by carboxylic extraction of rat heart atria, but as the amino-acid sequences were developed, most ANF peptides may now be produced synthetically. In the carboxylic acid extraction rat atria, either freshly dissected or frozen were obtained from standard commercial sources (i.e. diet of the rat is unimportant), finely ground in liquid nitrogen and extracted with a mixture of acetic acid and HCl containing phenyl methyl sufonyl fluoride in a homogenizer. After centrifugation the supernatant was passed through Sep-Pak cartridges and the cartridges were washed with trifluoroacetic acid (TFA) and eluted with acetonitrite in TFA. the eluates were reduced in volume and again separated on a $C_{18}$ column. The column was eluted with acetonitrite at different gradients.

Radioimmunoassays for ANF have also been developed in conventional manner by injection of chemically synthesized peptide, coupled to thyroglobulin, into rabbits for production of specific antisera. The antisera has then been combined into specific RIAs.

In the present specification the symbols for amino acids are according to standard IUPAC-IUB recommendations and single-letter symbols and three-letter symbols are used interchangeably for convenience according to the following table:

A Ala Alanine
B Asx Asparagine or aspartic acid
C Cys Cysteine
D Asp Aspartic acid
E Glu Glutamic acid
F Phe Phenylalanine
G Gly Glycine
H His Histidine
I Ile Isoleucine
K Lys Lysine
L Leu Leucine
M Met Methionine
N Asn Asparagine
P Pro Proline
Q Gln Glutamine
R Arg Arginine
S Ser Serine
T Thr Threonine

V Val Valine
W Trp Tryptophan
Y Tyr Tyrosine
Z Glx Glutamine or glutamic acid

## Summary of the Invention

During the course of experiments into ANF, and in particular cardionatrin extracted in conventional manner as briefly described above from rat atria it was noted that certain protein fractions eluting at about 33% $CH_3CN$ somewhat different diuretic, natriuretic and hypotensive responses to those normally associated with ANF. These fractions were, therefore purified to homogeneity by successive gradient-chromatography using TFA, hepta-fluorobutyric acid (HFBA) and ammonium acetate, and have been shown to have an entirely different composition to ANF. We have isolated a peptide, a 46 amino acid residue sequence:
Ser-Gln-Asp-Ser-Ala-Phe-Arg-Ile-Gln-Glu-Arg-Leu-Arg-Asn-Ser-Lys-Met-Ala-His-Ser-Ser-Ser-Cys-Phe-Gly-Gln-Lys-Ile-Asp-Arg-Ile-Gly-Ala-Val-Ser-Arg-Leu-Gly-Cys-Asp-Ile-Leu-Leu-Ile-Ala-Gln.

This sequence has a disulfide double bond between cysteine residues. This sequence, hereinafter designated iso-rANF$_{(1-46)}$ has not been found in a search in the Protein Data Banks of the National Library of Medicine and is, therefore, believed to be a novel peptide. The diuresis and natriuresis effects noted for this peptide are not due to the co-incident presence of ANF (ANP) because sequence analysis of the purified peptide does not reveal any ANP or pro-ANP sequence. It is another object to synthesize a peptide corresponding to residues 17-46 of iso-rANP$_{(1-46)}$ including the disulfide bond, which is both analogous and homologous to ANP$_{(99-126)}$ and to the recently discovered pig brain natriuretic peptide (BNP). This peptide, hereinafter designated iso-rANP$_{(17-46)}$, elicits diuretic, natriuretic, hypotensive and bradycardic responses qualitatively similar to both the native peptide iso-rANP$_{(1-46)}$ and rat ANP (hereinafter r-ANP), when injected into bioassay rats. Thus iso-rANP$_{(17-46)}$ has an amino acid residue sequence comprising:

```
      ┌──────S-S──────┐
MAHSSCFGQKIDRIGAVSRLGCDILLIAQ.
```

It is another object of this invention to provide a radioimmune assay and kit for detecting the 1-20 amino acid sequence of iso-rANP$_{(1-46)}$.

## Brief Statement of Invention

By one aspect of this invention there is provided a peptide in substantially pure form selected from the group of peptides having the amino acid sequence $R_1$-Ser-Ser-Cys-Phe-Gly-Glu-Lys-Ile-Asp-Arg-Ile-Gly-Ala-Val-Ser-Arg-Leu-Gly-Cys-Asp-$R_2$-$R_3$ and a disulphide double bond between said Cys and wherein $R_1$ is H, Ser, His-Ser, Ala-His-Ser, Met-Ala-His-Ser or Ser-Gln-Asp-Ser-Ala-Phe-Arg-Ile-Gln-Glu-Arg-Leu-Arg-Asn-Ser-Lys-Met-Ala-His-Ser, $R_2$ is Ile, Ile-Leu, Ile-Leu-Leu, Ile-Leu-Leu-Ile, Ile-Leu-Leu-Ile-Ala or Ile-Leu-Leu-Ile-Ala-Gln and $R_3$ is OH or $NH_2$.

By a preferred aspect of this invention there is provided a peptide having an amino acid sequence:
Ser-Gln-Asp-Ser-Ala-Phe-Arg-Ile-Gln-Glu-Arg-Leu-Arg-Asn-Ser-Lys-Met-Ala-His-Ser-Ser-Ser-Cys-Phe-Gly-Gln-Lys-Ile-Asp-Arg-Ile-Gly-Ala-Val-Ser-Arg-Leu-Gly-Cys-Asp-Ile-Leu-Leu-Ile-Ala-Gln,
with a disulfide bond between cysteine residues.

By another preferred aspect of this invention there is provided a peptide having an amino acid sequence:
Met-Ala-His-Ser-Ser-Ser-Cys-Phe-Gly-Glu-Lys-Ile-Asp-Arg-Ile-Gly-Ala-Val-Ser-Arg-Leu-Gly-Cys-Asp-Ile-Leu-Leu-Ile-Ala-Gln, with a disulphide bond between the Cys residues.

By another aspect of this invention there is provided a radioimmune assay and kit therefor, said kit comprising an antiserum to the 1-20 amino acid sequence of iso-rANP$_{(1-46)}$, an antigen consisting of a synthetic iso-rANP$_{(1-20)}$ having an added Tyr residue at the amino terminal serine (hereinafter iso-rANPY$_{(1-20)}$.

Brief Description of Drawings

Fig 1(a) is a biogel P10 chromatogram of lyophilized rat atrial extract for iso-rANP$_{(1-46)}$;

Fig 1(b) is a chromatogram for the first reverse phase HPLC purification of the extract from Fig 1(a);

Fig 1(c) is a chromatogram for the second reverse phase HPLC purification of the extract from Fig 1-(a);

Fig 1(d) is a chromatogram for the third reverse phase HPLC purification of the extract from Fig 1(a);

Fig 2A is a graph illustrating repetitive yield of phenylthiohydantoin (PTH) amino acids liberated at each cycle of Edman degradation of reduced pyridyelethylated iso-rANP$_{(1-46)}$;

Fig 2B is a sketch illustrating alignment of iso-rANP$_{(1-46)}$ with pro-ANP; and

Fig 2C is a schematic comparison of the sequences of iso-rANP$_{(17-46)}$, ANP$_{(99-126)}$ and BNP showing the large amount of homology which exists between all three in the vicinity of the disulfide ring.

Detailed Description of Preferred Embodiments

As noted above ANP$_{(99-126)}$ is the major circulating form of ANF and exerts its hormonal effects at receptor sites in kidney, smooth muscle, adrenal and other tissues. At least two types of ANP receptors have been identified. One ANP receptor is linked to particulate guanylate cyclase and mediates its action through cGMP while the other is not linked to guanylate cyclase and appears to be biologically silent. It has been suggested that the latter serves as a storage-clearance binding site for the hormone. Occupation of this so-called 'silent receptor' by a ring-depleted analogue of ANP causes an elevation in endogenous plasma ANP. This analogue is devoid of agonist and antagonist activity on the generation of cGMP but it competes effectively with biologically active atrial peptides for binding sites. Thus, an alternative to the storage-clearance role hypothesized for these receptors is that the 'silent' receptors are responsive to ANP-like peptides rather than to ANP itself.

The present peptides have resulted from this hypothesis. They are not related to ANP but have similar biological action. In the initial stages of the present development a peptide which co-eluted with a truncated form of pro-ANP on reverse phase HPLC of a rat atria extract was isolated and purified during the routine purification of pro-ANP and ANP, and when injected into assay rats, gave rise to a diuresis and natriuresis. This effect was not due to the co-incident presence of ANP because sequence analysis of the purified peptide did not reveal any ANP or pro-ANP sequence. When sequenced by an automated gas phase sequencer the following amino acid sequence for the first 20 cycles of the sequencer was obtained: Ser-Gln-Asp-Ser-Ala-Phe-Arg-Ile-Gln-Glu-Arg-Leu-Arg-Asn-Ser-Lys-Met-Ala-His-Ser. A search of the Protein Data Bank of the National Biomedical Research Foundation revealed that this was a previously unknown sequence. The small amounts of this peptide that were available precluded extensive sequence analysis but amino acid composition of the peptide suggested a longer peptide. This, together with the biological activity and chemical uniqueness of the sequenced portion, indicated that the sequence was the amino terminal end of a new diuretic/natriuretic peptide.

Chemical synthesis of the 20 amino acid fragment was effected and anti-sera was raised in rabbits for the purpose of developing a specific radioimmunoassay (RIA).

The synthesis was conducted using standard solid-phase synthesis on a model 430 Applied Biosystems Peptide Synthesizer. The peptide was cleaved from the resin with hydrogen fluoride. Peptides were purified by semipreparative high performance liquid chromatography (HPLC) using a Vydac $C_{18}$ column. The composition and sequence of each peptide was verified by amino acid analysis on a Beckman 6300 automatic Amino Acid Analyzer and by gas-phase sequence analysis (Applied Biosystems Model 470A sequencer with on-line phenylthiohydantoin (PTH) analysis).

The procedure was repeated to produce the same 20 amino acid residue fragment with a tyrosine residue attached to the N-terminal serine. The 20-residue peptide (P2A) was mixed with Freund's complete adjuvant and injected subcutaneously into New Zealand white rabbits. After boosting at 5 weeks after the initial injection the rabbits were bled 1 week later. The anti-serum gave precipitin lines with its homologous antigen and with the native peptide extracted from atria when examined by double immunodiffusion. No cross reactivity under these conditions occurred with ANP$_{(99-126)}$, ANP$_{(101-126)}$ or with pro-ANP. Tyrosinated P2A (P2A-Y) was iodinated using the chloramine T method and the iodinated tracer was purified by HPLC on a Waters µBondapak $C_{18}$ column. For radioimmunossay (RIA) the buffer contained 0.1M $NaHPO_4$ buffer, pH 7.4, 0.05M NaCl, 0.1% BSA and 0.02% $NaCl_2$. The RIA incubation mixture contained 3-100 pg of P2A in 100 µ l of buffer, 100 µl of diluted antiserum and 100 l of $^{125}$I-P2A-Y (approx. 8000 cpm). After incubation overnight at 4°C bound and free iodinated peptide were separated by second antibody precipitation using goat anti-rabbit - globulin. The sensitivity of the assay was 2-3 pg/tube with 50% displacement at 25-30 pg/tube.

This antiserum did not cross-react with ANP or with pro-ANP. The RIA was then used to monitor the purification of the peptide de novo from atrial extracts using gel-filtration and reverse-phase HPLC. The procedure used was essentially the same as the one briefly described above for the purification of ANP, except that the initial extraction and the gel-filtration stages were done in the presence of several protease inhibitors.

900 frozen rat atria (Rockland, Inc.) were ground in liquid nitrogen and homogenized using a polytron in 6 volumes (by weight) of 1M aoetic acid, 1M HCl, 2.5mM PMSF, 1mM EDTA, 20 µg/ml each of leupeptin, pepstatin and aprotinin. After centrifugation the supernatant was filtered through wetted gauze and the pellet was rehomogenized in 4 volumes (by weight) (total of 10 volumes) of homogenizing buffer. After centrifugation the supernatant was filtered through gauze and pooled with supernatant from the first centrifugation. The supernatants (15 mL portions) were passed through wetted Sep Pak (Waters) cartridges. The effluent was collected (30 ml) and passed through a second Sep Pak cartridge. Each cartridge was washed with 10 ml 0.1% TFA and eluted with 3 ml 80% acetonitrile in 0.1% TFA. All eluted material was combined and lyophilized.

Lyophilized atrial extract was dissolved in 5ml 1M acetic acid containing 1% NaCl and loaded onto a Biogel P10 column (2.5 cm x 40 cm) equilibrated with 1M acetic acid in 1% NaCl. The column was eluted isocratically at a flow rate of 0.8 ml/min. Absorbance, at 280 nm, of each fraction was determined and an RIA was performed using an aliquot of each fraction. Immunoreactive (IR) peak fractions 37-53 as shown in Fig 1(a) were collected and dialyzed against 0.1% trifluoroacetic acid (TFA) (5L).

The IR fraction from Biogel P10 was applied to a Bydac $C_{18}$ semipreparative column (10 mm x 250 mm) equilibrated with 0.1% TFA. The column was eluted at a flow rate of 3 ml/min using a gradient of acetonitrile. The immunoreactive fraction coeluated with the peak at fractions 41-43 as shown in Fig 1(b).

The IR faction was further purified on a Vydac $C_{18}$ analytical column (4.6 mm x 250 mm) with a gradient of acetonitrile in the presence of 0.1% HFBA. IR peak as shown in Fig 1(c) was collected in fractions 31-33.

The IR peak was then applied to a Vydac $C_{18}$ (4.6 mm x 250 mm) column and eluted with a gradient of acetonitrile in 0.1% TFA. IR iso-rANP as shown in Fig 1(d) corresponds to the peak.

The finally purified peptide appeared homogeneous by SDS gel electrophoresis, which indicated a molecular weight of about 5500. From the RIA it was estimated that the atria contain 0.7 ng/mg of iso-rANP which is considerably less than the value of about 175 ng/mg calculated for immunoreactive (IR) ANP in atria. The amino acid composition of the peptide was Cys-COOH (1.6); Asx (4.5); Thr (1.1); Ser (7.1); Gln (4.5); Gly (4.0); Ala (3.0); Met (0.84); Ile (4.72); Leu (3.97); Phe (1.85); His (0.9); Lys (2.2); Arg (5.7); indicating that the peptide was composed of 44-46 residues and contained two Cys residues. For sequencing, the peptide was reduced and pyridylethylated then subjected to sequencing, using a gas-phase automatic sequencer, and the complete amino acid sequence of the peptide was elucidated (Fig 2A). Positive confirmation of the deduced structure containing a disulfide bond was performed by chromatographic comparison of enzymatically cleaved reduced and non-reduced native peptide and an identically sequenced synthetic peptide containing an intramolecular disulfide bond.

Thus, it is clear that the preliminary 20 amino acid fragment, iso-rANP$_{(1-20)}$ constitutes the amino terminal end of a longer 46 residue peptide (Fig 2A). The 46 amino acids of this peptide maximally align with C-terminal portion of pro-ANP, with striking homology exhibited within the disulfide bonded ring (Fig 2B).

Little homology is shown between residues 1-20 of iso-rANP$_{(1-46)}$ and the amino terminal extension of ANP$_{(99-126)}$ i.e. residues 83-98 of pro-ANF. However, Lys 16 of iso-rANP$_{(1-46)}$ is homologous with Arg 98 of pro-ANP and represents a possible cleavage site. Residues 17-46 of iso-rANP$_{(1-46)}$ form a putative peptide which is both analogous and homologous to ANP$_{(99-126)}$ and to the recently discovered pig brain natriuretic peptide (BNP; Fig. 2C).

The iso-rANP$_{(17-46)}$, containing a disulfide linkage between the two Cys residues, was chemically synthesized using the method noted above.

When injected into bioassay rats, the synthetic peptide elicited diuretic, natriuretic, hypotensive and bradycardic responses qualitatively similar to both the native peptide and rANP (Table 1).

**TABLE 1**

Peak cardiovascular and renal responses induced by I.V. bolus injections of native iso-rANP$_{(1-46)}$, iso-rANP$_{(17-46)}$ and rANP$_{(99-126)}$. Assay rats anesthetized with sodium pentobarbital and given a constant infusion of saline. All peak responses occurred within 10 min of injection. (average % change ± SEM)

| | Vehicle (0.2ml) | iso-rANP$_{(1-46)}$ | iso-rANP$_{(17-46)}$ | | | rANP$_{(99-126)}$ |
|---|---|---|---|---|---|---|
| Dose (nanomol) | 0 (6) | 0.5* (4) | 0.8 (6) | 1.5 (6) | 3.0 (12) | 0.7 (6) |
| % Decrease | | | | | | |
| MAP | -3.3 ± 3.3 | -17.1 ± 4.5 | -7.2 ± 2.0 | -22.7 ± 11.0 | -18.7 ± 3.0 | -22.8 ± 4.1 |
| HR | -2.5 ± 3.1 | - 8.9 ± 2.5 | -5.7 ± 2.0 | -11.9 ± 3.0 | - 6.9 ± 1.8 | -10.3 ± 1.2 |
| % Increase | | | | | | |
| Urine Volume | +8 ± 20 | +448 ± 230 | +124 ± 102 | + 233 ± 151 | + 418 ± 169 | + 687 ± 164 |
| $Na^+$ Excretion | +2 ± 21 | +592 ± 251 | +178 ± 137 | +1220 ± 1008 | +1073 ± 526 | +1574 ± 542 |
| $K^+$ Excretion | +3 ± 17 | +545 ± 229 | + 85 ± 73 | + 175 ± 151 | + 247 ± 101 | + 440 ± 157 |
| $Cl^-$ Excretion | +8 ± 18 | +344 ± 145 | + 89 ± 74 | + 292 ± 216 | + 548 ± 236 | + 697 ± 183 |

Vehicle, 0.01M acetic acid, SEM, standard error of the mean; n in brackets is number of assay rats; MAP, mean arterial pressure; HR, heart rate.

*Based on a molecular weight of approximately 5,500.

Experiments were performed using male Sprague-Dawley rats, ranging in weight from 270-362 g. The rats were anesthetized by an intraperitoneal injection of sodium pentobarbital (70 mg.kg-1; MTC Pharmaceuticals, Hamilton, Ont.). Additional small amounts of anesthetic were administered as required in the course of the experiment. Body temperature was maintained by a heating lamp and rectal temperature monitored by a rectal prove. After installing a tracheal cannula, a femoral vein was cannulated and a priming infusion of saline (0.9% NaCl) was administered over a 20 min period (1.1 ml); this was followed by a maintenance saline infusion of 1.2 ml.h-1. The femoral artery was then cannulated for blood pressure measurement (Statham pressure transducer, model P23Db). A catheter was also advanced via the right external jugular vein towards the right heart for bolus injections of vehicle, ANF or the atrial peptide of the present invention (AHP). After tying off the penis, the bladder was cannulated for urine collection. Rates were not used or experiments aborted if the urine became bloody. The EKG was recorded for determination of heart rate.

Urine was collected in preweighed vials and volume calculated by weighing. The time for sequential urine collections was 5 min or, increments of 4 min until there was enough urine volume for electrolyte determinations (0.25 ml or 16 drops). Rat kidneys were removed at the termination of the experiments so that urinary excretion measurements could be normalized for total kidney weight. Measurements of NA+ and K+ were obtained using ion specific electrodes and Cl- was obtained by coulometric titration.

Zero time was established by 40-60 mins from the beginning the priming saline infusion. The control measurements were carried out over 20 mins or, until there was sufficient urine collection for electrolyte determinations. A bolus injection of vehicle, $rANP_{(99\text{-}126)}$ or iso-$rANP_{(1\text{-}46)}$ or iso-$rANP_{(17\text{-}46)}$ was then administered into the jugular vein catheter and the catheter immediately cleared of remaining injectate by a secondary bolus of 0.2 ml saline. The $rANP_{(99\text{-}126)}$ was obtained from Armand-Frappier, Montreal. Cardiovascular measurements were analyzed at 5 min intervals during control and at 1, 5, 10, 15 and 20 mins following administration of injectate. Injection of reduced pyridyllethylated iso-$rANP_{(1\text{-}46)}$ or iso-$rANP_{(17\text{-}46)}$ in the same concentration shown in Table 1 did not elicit a biological response. Thus, residues 17-46 of the native peptide represent a functional, as well as structural, homologue of both ANP and BNP. Moreover, it would appear that residues 1-20 of iso-$rANP_{(1\text{-}46)}$ are not required for biological activity. Also, synthetic iso-$rANP_{(1\text{-}20)}$ did not have any intrinsic biological activity. As shown in Table 1, the potency of iso-$rANP_{(17\text{-}46)}$ is somewhat less than $rANP_{(99\text{-}126)}$. It required twice the dose of iso-$rANP_{(17\text{-}46)}$ (1.5 nanomol) to produce equivalent average decreases in mean arterial blood pressure and heart rate as $rANP_{(99\text{-}126)}$ (0.7 nanomol). Furthermore, increasing the dose of iso-$rANP_{(17\text{-}46)}$ to 3.0 nanomol has no additional effect on these cardiovascular responses. With respect to urinary responses, at a dose level where rANP responses are maximal (0.7nmol), 0.9 nmol of iso-$rANP_{(17\text{-}46)}$ had only a small effect in most rats. A four-fold larger dose of iso-$rANP_{(17\text{-}46)}$ (3.0 nanomol) than $rANP_{(99\text{-}126)}$ had a somewhat lesser effect in increasing urinary volume as well as $Na^+$, $K^+$ and $Cl^-$ excretion. Variability of response of assay rats to injected peptides is evident from the rather larger variances (SEM).

Both iso-$rANP_{(17\text{-}46)}$ and the native peptide, like $rANP_{(99\text{-}126)}$, showed smooth muscle vaso-relaxant activity.

Aortic rings (3-4mm) were cut from the thoracic aortae of New Zealand White rabbits. The rings were suspended in a 10 ml tissue bath containing Kreb's Ringer Solution, pH 7.4 at 37° C. The rings were maintained at about 70% of maximal contraction with phenylephrine in doses ranging from $1 \times 10^{-6}$ to $5 \times 10^{-8}$M. Vasorelaxant activity or peptides was tested by adding various concentrations of peptide to the bath. Native iso-$rANP_{(1\text{-}46)}$ caused a slower and smaller relaxation of phenylephrine induced contraction of the rabbit throacic aortal rings compared to $rANP_{(99\text{-}126)}$.

The synthetic iso-$ANP_{(17\text{-}46)}$ was also less potent than $ANP_{(99\text{-}126}$. A 20-fold higher dose was required to produce the same relaxation obtained by 50 ng of $rANP_{(99\text{-}126)}$. Reduced pyridyllethylated iso-$rANP_{(1\text{-}46)}$ and iso-$rANP_{(17\text{-}46)}$ were devoid of smooth muscle vaso-relaxant activity.

Iso-rANP elicits weaker biological responses than rANP, but it is remarkable that it produces the biological responses it does, since the C-terminal portion of the peptide does not contain the Phe-Arg-Tyr sequence, which appear to be essential in rANP for maximal activity. This C-terminal sequence of rANP is also retained in BNP (Fig 2C). The amino terminal extension of iso-$rANP_{(17\text{-}46)}$ bears little sequence homology to the amino terminal extension of $ANP_{(99\text{-}126)}$. The structure of the native peptide suggests that iso-rANP is, like rANP, derived from a larger precursor, which is genetically distinct from both ANP and BNP.

The existence of both iso-rANP and BNP indicates that there is a family of natriuretic peptides. BNP has only been found in brain, where it may act in concert with ANP in the control of water intake and salt appetite. Since a number of peptides originally discovered in brain have been shown to be present in other organs, it is possible that BNP occurs in other tissues also. Iso-rANP indicates that at least two natriuretic peptides are made by the heart. Iso-rANP may function in concert with rANP in regulating electrolyte and fluid balance and blood pressure and may compete for rANP kidney receptors. At least two kidney receptors for ANP have been demonstrated, one of which may be biologically silent.

## Claims

1. A peptide in substantially pure form selected from the group of peptides having the amino acid sequence: $R_1$-Ser-Ser-Cys-Phe-Gly-Glu-Lys-Ile-Asp-Arg-Ile-Gly-Ala-Val-Ser-Arg-Leu-Gly-Cys-Asp-$R_2$-$R_3$ and a disulphide double bond between said Cys and wherein $R_1$ is H, Ser, His-Ser, Ala-His-Ser, Met-Ala-His-Ser or Ser-Gln-Asp-Ser-Ala-Phe-Arg-Ile-Gln-Glu-Arg-Leu-Arg-Asn-Ser-Lys-Met-Ala-His-Ser, $R_2$ is Ile, Ile-Leu, Ile-Leu-Leu, Ile-Leu-Leu-Ile, Ile-Leu-Leu-Ile-Ala or Ile-Leu-Leu-Ile-Ala-Gln and $R_3$ is OH or $NH_2$.

2. A peptide as claimed in claim 1 wherein $R_1$ is Met-Ala-His-Ser and $R_2$ is Ile-Leu-Leu-Ile-Ala-Gln.

3. A peptide as claimed in claim 1 wherein $R_1$ is Ser-Gln-Asp-Ser-Ala-Phe-Arg-Ile-Gln-Glu-Arg-Leu-Arg-Asn-Ser-Lys-Met-Ala-His-Ser and $R_2$ is Ile-Leu-Leu-Ile-Ala-Gln.

4. A therapeutic composition of matter for producing natriuresis and diuresis, when injected into a non-diuretic rat, comprising a therapeutically effective amount of the peptide of Claim 1 and a pharmaceutically acceptable carrier.

5. A therapeutic composition of matter for producing vascular smooth muscle relaxant activity comprising a therapeutically effective amount of the peptide of claim 1 and a pharmaceutically acceptable carrier.

6. A method of causing a diuretic, natriuretic, vasorelaxant or hypotensive response in a patient, comprising administering to said patient an effective amount of a peptide as claimed in claim 1.

7. A diagnostic radioimmunoassay kit comprising:
An antiserum to a 1-20 amino acid sequence of iso-$rANP_{(1-46)}$, a synthetic peptide antigen consisting of iso-$rANP_{(1-20)}$, and a peptide consisting of iso-$rANP_{(1-20)}$ having Tyr added to the amino terminal serine thereof.

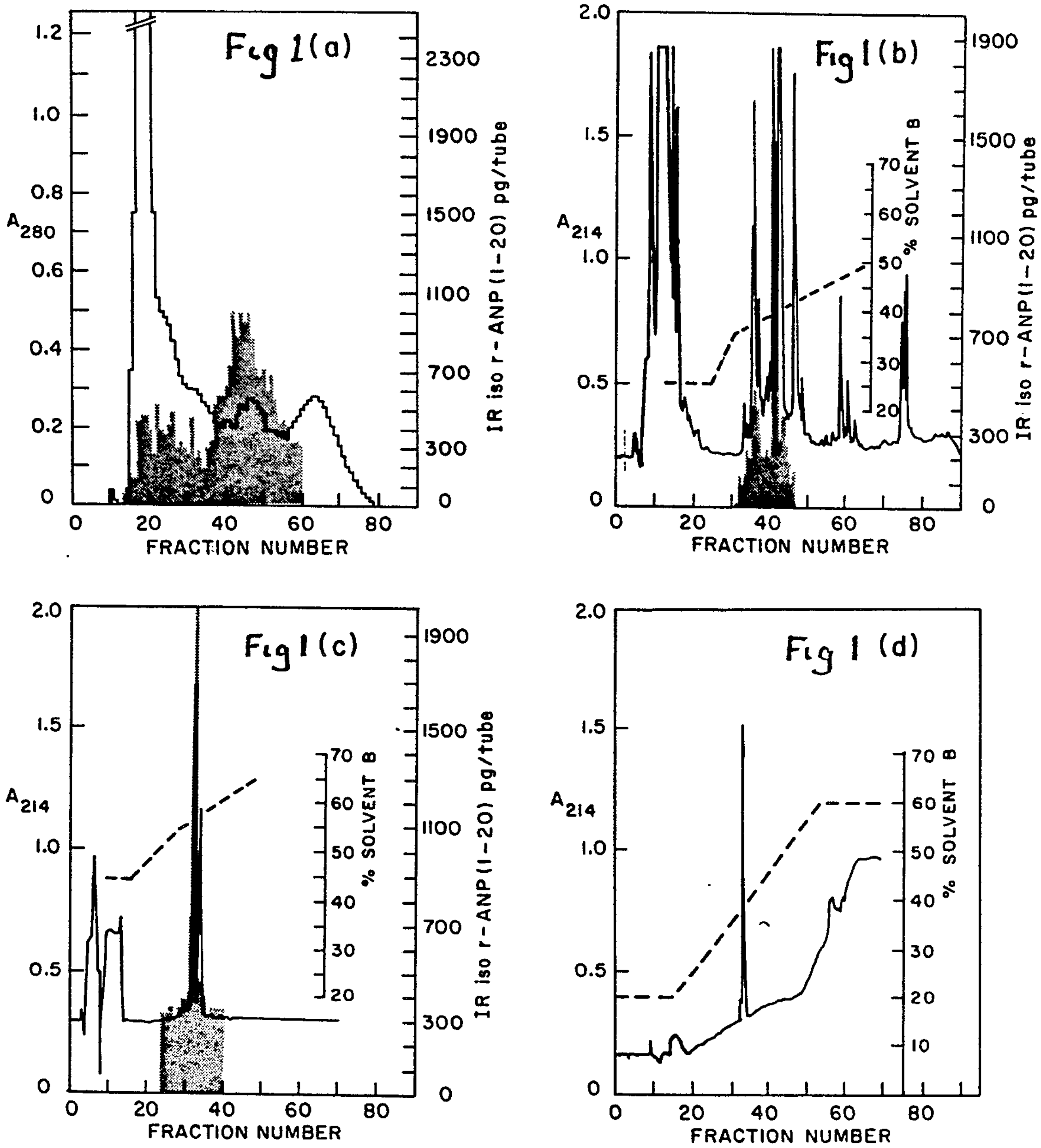
Fig 1(a)
A280
IR iso r-ANP (1-20) pg/tube
FRACTION NUMBER
Fig 1(b)
A214
% SOLVENT B
IR iso r-ANP (1-20) pg/tube
FRACTION NUMBER
Fig 1(c)
A214
% SOLVENT B
IR iso r-ANP (1-20) pg/tube
FRACTION NUMBER
Fig 1 (d)
A214
% SOLVENT B
FRACTION NUMBER

FIG 2

A

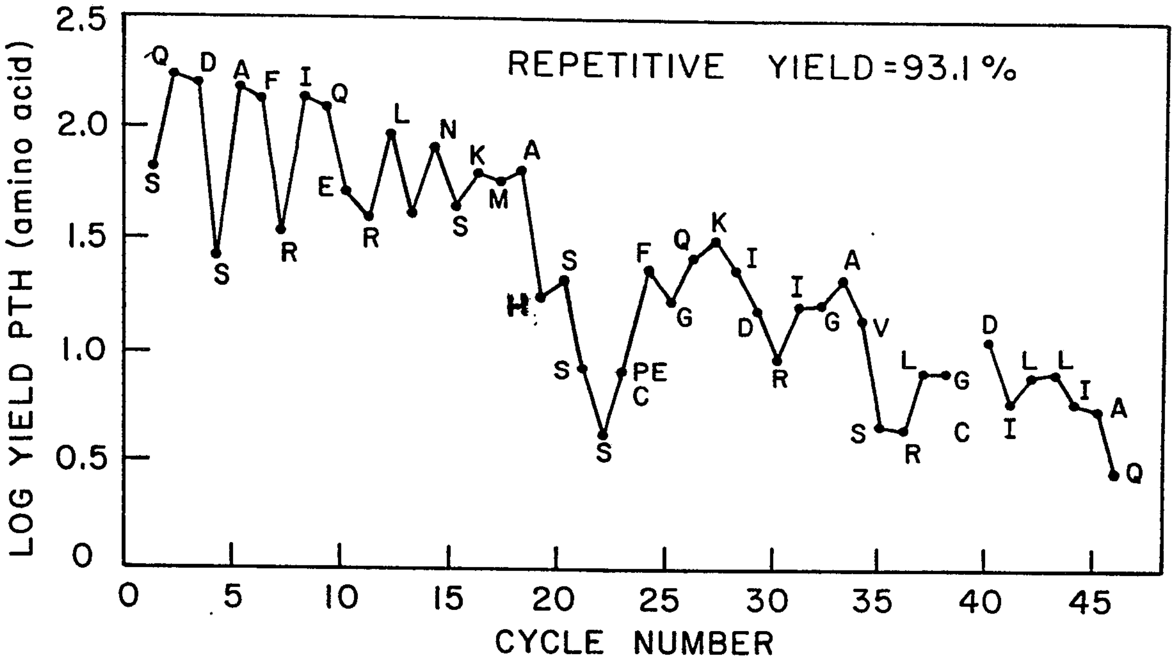


B

```
                 1                 10                  20                  30
pro-rANP         N P V Y S A V S N T D L M D F K N L L D H L E E K M P V E D

                                   40                  50                  60
                 E V M P P Q A L S E Q T D E A G A A L S S L S G V P P W T G

                                   70                  80                  90
                 E V N P S Q R D G G A L G R G P W D P S D R S A L L K S K L
iso-rANP(1-46)                                         S Q D S A F-------- R I
                                                       1                   7

                                   100                 110                 120
                 R A L L A G P R S L R R S S C F G G R I D R I G A Q S G L G
                 Q E R L R N S K M A H S S S C F G Q K I D R I G A V S R L G
                   10                  20                  30

                           126
                 C N S F R Y
                 C D I L L I A Q
                   40      46
```

C

```
iso-rANP(17-46)   M A H S S S C F G Q K I D R I G A V S R L G C D I L L I A Q
rANP(99-126)    S L R R S S C F G G R I D R I G A Q S G L G C N S F   R Y
BNP                   D S G C F G R R L D R I G S L S G L G C N V L R R Y
```